Europäisches Patentamt

European Patent Office

Office européen des brevets

(11) Numéro de publication: **0 316 212 B1**

(12)
# FASCICULE DE BREVET EUROPEEN

(45) Date de publication de fascicule du brevet: **08.03.95** (51) Int. Cl.6: **A61K 39/395**, A61K 31/06, A61K 47/00

(21) Numéro de dépôt: **88402733.5**

(22) Date de dépôt: **28.10.88**

(54) **Application de nitrophényles ou d'anticorps anti-nitrophényles à la modification de l'interaction entre des cellules sensibles d'un hôte et un agent pathogene.**

(30) Priorité: **30.10.87 FR 8715126**

(43) Date de publication de la demande:
**17.05.89 Bulletin 89/20**

(45) Mention de la délivrance du brevet:
**08.03.95 Bulletin 95/10**

(84) Etats contractants désignés:
**AT BE CH DE ES FR GB GR IT LI LU NL SE**

(56) Documents cités:
**EP-A- 0 008 092
EP-A- 0 068 763**

**JOURNAL OF IMMUNOLOGY, vol. 124, no. 2, février 1980, The Williams & Wilkins Co.; Z. ESHHAR et al., pp. 775-780&NUM;**

**BIOLOGICAL ABSTRACTS, vol. 83, no. 5, 1987, Philadelphia, PA (US); P.R. BEINING et al., no. 44249&NUM;**

**CHEMICAL ABSTRACTS, vol. 90, no. 23, 04 juin 1979, Coulumbus, OH (US); D. INBAR et al., p. 482, no. 184730a&NUM;**

(73) Titulaire: **INSTITUT PASTEUR
25-28, rue du Docteur Roux
F-75724 Paris Cédex 15 (FR)**

(72) Inventeur: **Avrameas, Eustrate-Stratis
1 rue Sarasate
F-75015 Paris (FR)**
Inventeur: **Matsiota-Bernard, Panayota
12 Square Beethoven
F-78330 Fontenay-Le-Fleury (FR)**
Inventeur: **Ternynck, Thérèse
39 bld Garibaldi
F-75015 Paris (FR)**
Inventeur: **Saron, Marie-Françoise
185 bld V. Auriol
F-75013 Paris (FR)**
Inventeur: **Guillon, Jean-Claude
5 Résidence du Parc
F-91120 Palaiseau (FR)**
Inventeur: **Montagnier, Luc
21 rue Malabry
F-92350 Le Plessis-Robinson (FR)**
Inventeur: **Guetard, Denise
4 bis, rue Anselm Payen
F-75015 Paris (FR)**

Rank Xerox (UK) Business Services
(3.10/3.09/3.3.3)

**PATENT ABSTRACTS OF JAPAN, vol. 6, no. 139 (C-116)(1017), 28 juillet 1982&NUM;**

Inventeur: **Favier, Véronique**
**2 rue Coriolis**
**F-75012 Paris (FR)**

(74) Mandataire: **Gutmann, Ernest et al**
**Ernest Gutmann - Yves Plasseraud S.A.**
**3, rue Chauveau-Lagarde**
**F-75008 Paris (FR)**

**Description**

La présente invention a pour objet un procédé de modification des conditions d'interaction entre des cellules sensibles d'un hôte et un agent pathogène. D'un autre point de vue, elle a pour objet l'application de nitrophényles et de leurs dérivés ou d'anticorps anti-nitrophényles polyclonaux ou monoclonaux, à la modification de l'interaction entre des cellules sensibles d'un hôte et un agent pathogène.

Par cellule sensible on entend toute cellule de l'hôte capable d'être affectée de façon pathologique par un agent pathogène, l'effet pathologique résultant notamment de l'introduction de l'agent pathogène dans ces cellules ou de sa fixation sur ces cellules par exemple sur un récepteur approprié de celles-ci.

Des cellules sensibles constituant des cibles pour certains agents pathogènes sont notamment les lymphocytes T ou B, ou les thymocytes, les fibroblastes, les cellules rénales etc...

L'agent pathogène en cause est souvent un micro-organisme, par exemple un virus, ou une molécule ou un agrégat de molécules capable d'interférer avec le métabolisme cellulaire, lorsqu'il pénètre dans les cellules sensibles définies ci-dessus ou réagit avec celles-ci de toute autre manière, par exemple lorsque l'agent pathogène est un auto-anticorps.

Il a déjà été observé à l'occasion de l'interaction in vivo entre un agent pathogène, par exemple un virus infectieux et les cellules sensibles, une modification du taux d'anticorps naturels (AcN) chez l'hôte atteint de la maladie infectieuse ou autoimmune correspondante. Ces AcN sont des anticorps contenus dans les sérums et les liquides biologiques des humains et des animaux, capables de reconnaître des antigènes (Ag) du "soi" et du "non-soi". Ces AcN existent dans les liquides biologiques alors même que les hommes et les animaux n'ont pas été préalablement intentionnellement immunisés contre un agent pathogène déterminé.

La polyspécificité des AcN et le fait que souvent ils possèdent une haute affinité pour un antigène donné (T. Ternynck, S.Avrameas, Immunol. Rev. 1986) leur permet de reconnaître plusieurs antigènes présents chez des agents pathogènes. Il est à noter par ailleurs que parmi ces AcN, certains possèdent une activité du type anti-dinitrophényle (DNP) ou anti-trinitrophényle (TNP) et que dans plusieurs situations pathologiques (maladies autoimmunes et infections virales) le titre de ces anticorps naturels anti-NP sériques est significativement modifié (P. Matsiota et al. Clin. exp. Immunol., 1987, et Ann. Inst. Pasteur 1987).

La publication de Beining P.R. et al (Transplantation, 1986, vol. 42, n° 5, p. 524-528) décrit des tests effectués sur des souris, visant à déterminer l'effet d'anticorps monoclonaux anti-DNP administrés préalablement à l'injection de DNP, sur la réponse humorale de ces souris.

Comme l'ont remarque les auteurs, ces tests sont effectués dans le contexte d'une hypothèse d'utilisation dans le cadre de transplantation en vue d'améliorer la survie du greffon.

Pour réaliser un exemple, ces auteurs ont utilisé le couple DNP/anticorps anti-DNP.

Les anticorps anti-DNP utilisés parallèlement au DNP, représentent ici exclusivement un outil pour évaluer un effet d'immunosuppression, sans qu'un autre intérêt des anticorps ait été divulgué.

Pour des commodités de langage, on regroupera sous le terme NP, les molécules contenant des molécules contenant un cycle benzénique dont un, deux ou trois atomes d'hydrogène est ou sont substitué-(s) par un groupe nitro (NO$_2$), de préférence 2 ou 3 groupes nitro. Des molécules NP préférées sont des nitrophénols. D'autres molécules intéressantes sont plus particulièrement le 2,4-dinitrophényle (DNP), ou le 2,4,6-trinitrophényle (TNP).

On a plus particulièrement remarqué que le taux d'anticorps naturels ayant une activité anti-NP (AcN anti-NP) est significativement plus élevé que le taux des autres anticorps naturels lors de ces mêmes maladies auto-immunes ou de ces infections virales. Ces résultats ont notamment fait l'objet de publications à la suite d'études faites dans le cadre d'infections causées par le virus du SIDA (HIV)("Détection of natural auto-antibodies in the serum of anti-HIV-positive individuals.", autrement dit "Détection d'auto-anticorps naturels dans le sérum d'individus ayant une réaction positive anti-HIV". P.Matsiota et al. Ann. Inst. Pasteur/Immunol 1987, 138, 223-233.) et dans le cadre d'une étude concernant le lupus érythémateux ("Natural antibodies in systemic lupus erythematosus." autrement dit Anticorps naturels du lupus érythémateux de l'organisme". P. Matsiota et al. Clin. exp. Immunol (1987) 69, 79-88).

L'invention découle de la découverte faite par les inventeurs que la réalisation d'une mise en contact -in vitro ou in vivo- des cellules sensibles atteintes par l'agent pathogène ou susceptibles d'être atteintes, voire de l'agent pathogène lui-même, avec des anticorps anti-NP faisant l'objet d'un apport extérieur, d'une part, ou à l'inverse avec des dérivés de NP reconnus par des anticorps anti-NP, d'autre part, se manifeste par une modulation contrôlable des interactions entre lesdites cellules et ledit agent pathogène lorsque celui-ci et celles-là entrent en contact. Il est à remarquer que les effets induits par les anticorps anti-NP ou par les NP eux-mêmes sont souvent de même nature, et cela peut être parce qu'au plan immunologique, les NP peuvent être considérés comme des images internes des anticorps anti-NP.

L'invention vise donc un procédé pour modifier les conditions de l'interaction entre les cellules sensibles d'un hôte vivant et un agent pathogène pour cet hôte, caractérisé par la mise en contact de ces cellules sensibles à l'agent pathogène (in vitro ou in vivo) avec des anticorps anti-NP ou avec des NP ne possédant pas d'effets toxiques (on les appellera encore NP détoxifiés ou dérivés NP) et ce dans des conditions notamment de dosage des anticorps anti-NP ou des NP détoxifiés, fonction du degré de modulation recherché de la susdite interaction.

Le procédé selon l'invention est plus particulièrement applicable à la réalisation d'une modulation de l'interaction entre les cellules sensibles d'un hôte vivant et un agent pathogène consistant en un virus.

Par NP détoxifié (ou dérivé NP) on entend toute molécule NP modifiée chimiquement en C1 du cycle phényle ou au niveau de sa position phénol, par un groupe chimique assurant la neutralisation des propriétés toxiques in vivo de la molécule NP non modifiée. Cette modification chimique fait en particulier intervenir une liaison covalente entre la position phénol du NP et un groupe fonctionnel approprié, par exemple amino, du groupe de modification. Le groupe de neutralisation décrit ci-dessus est choisi de telle sorte qu'il n'affecte pas la capacité du NP à être reconnu par les anticorps anti-NP.

La nature de l'effet ou l'influence des anticorps anti-NP ou à l'inverse des NP détoxifés, sur l'interaction entre les cellules sensibles de l'hôte et l'agent pathogène à l'égard de ces cellules sensibles, peut être évaluée in vitro, en mettant en oeuvre un test comprenant :

- la mise en contact de l'agent pathogène avec des cellules sensibles atteintes ou susceptibles d'être atteintes par cet agent pathogène, en présence de doses variables, par exemple croissantes d'anticorps anti-NP ou de NP détoxifiés,
- l'observation pour chacune de ces doses de l'effet exercé par l'anticorps anti-NP ou le NP détoxifié sur l'interaction in vitro de l'agent pathogène et les cellules sensibles et
- l'appréciation de la variation de l'effet exercé sur cette interaction en fonction desdites doses.

On a en effet constaté que l'interaction entre l'agent pathogène et les cellules sensibles est en général modifiée en présence des anticorps anti-NP ou des NP détoxifiés, cette modification s'accompagnant dans la plupart des cas de l'incorporation d'une partie au moins des NP détoxifiés, aux cellules sensibles.

L'évolution de l'interaction entre l'agent pathogène et les cellules sensibles, lors de la mise en oeuvre du test précédent se manifeste, selon les cas et par exemple par :

- une inhibition de la fixation de l'agent pathogène sur les cellules sensibles ou de son internalisation dans ces cellules, éventuellement en raison d'un blocage possible des récepteurs cellulaires par les anticorps ou par les NP détoxifiés,
- un effet de neutralisation de la réplication (s'il s'agit d'un virus) ou de l'activité lytique de l'agent pathogène à l'égard des cellules sensibles, etc...

Ces effets peuvent subir des variations ou être inversés, en fonction des doses administrées.

L'évaluation in vitro des effets des anticorps anti-NP ou à l'inverse des NP détoxifiés sur l'interaction, entre les cellules sensibles de l'hôte et l'agent pathogène peut ainsi permettre l'orientation d'un traitement thérapeutique de l'individu atteint par un agent pathogène ou la prévention de la maladie correspondante chez cet individu, ce traitement thérapeutique comprenant alors l'administration à cet individu de doses appropriées d'anticorps anti-NP ou de NP détoxifiés.

La détermination des doses appropriées peut résulter de l'évaluation faite in vitro. Des adaptations de ces doses appropriées devront naturellement être réalisées par le clinicien, au vu des sensibilités différentes des patients aux anticorps anti-NP ou aux NP détoxifiés administrés.

Dans un mode de réalisation particulier du procédé précédent, la modification de l'interaction entre le virus et une cellule sensible à ce virus sera obtenue par la mise en contact de ces cellules avec un anticorps monoclonal anti-NP.

Ces anticorps peuvent notamment être obtenus au moyen de culture d'hybridomes réalisés par fusion de cellules de souris préalablement immunisées avec du NP et de cellules myélomateuses, ces hybridomes étant sélectionnés parmi les hybrides cellulaires résultant de ces fusions et produisant de tels anticorps monoclonaux.

La sélection des anticorps anti-NP pouvant être utilisés pour traiter un patient, selon le procédé de modification de l'interaction entre les cellules sensibles d'un hôte et un agent pathogène pour cet hôte, peut aussi être faite par mise en oeuvre du test d'évaluation de la modulation de l'interaction décrit plus haut en présence de ces anticorps monoclonaux, en particulier lorsqu'une première évaluation de cette modulation aura été faite au préalable avec des anticorps polyclonaux. La modulation observée peut être utilisée comme référence pour orienter la sélection des Ac-anti-NP actifs. Peuvent être retenus ceux des anticorps monoclonaux qui, dans des essais réalisés dans des conditions semblables à celles dans lesquelles les tests avec les anticorps polyclonaux avaient été réalisés, induisent des effets semblables ou plus intenses que ceux observés avec les anticorps polyclonaux.

Dans un autre mode de réalisation préféré les anticorps mis en oeuvre sont les anticorps monoclonaux naturels anti-NP.

Dans un autre mode de réalisation préféré de l'invention, on remplace les Ac-anti-NP par les NP détoxifiés. De façon préférée, le NP mis en oeuvre dans le cadre du procédé selon l'invention est chimiquement modifié en C1 du cycle phényle ou au niveau de sa position phénol par substitution par un acide aminé, par un peptide, par une protéine ou par tout groupe chimique détoxifiant, n'interférant et ne modifiant pas défavorablement l'influence exercée in vitro par les NP sur la susdite interaction.

Parmi les acides aminés particulièrement adaptés pour être substitués à la position phénol de la molécule NP, on citera particulièrement la glycine et la lysine.

Dans un mode de réalisation particulièrement préféré à ce jour du procédé selon l'invention, le NP sera modifié par la glycine.

Parmi les protéines adaptées à la mise en oeuvre du procédé selon l'invention, on peut mentionner la sérum-albumine-bovine (BSA)(abréviation anglaise de Bovine Serum-Albumin) l'ovalbumine (OVA) ainsi que la myosine.

Il va sans dire que ces molécules sont citées à titre d'exemples mais qu'elles ne limitent en rien le choix des groupes de substitution réalisant la modification de la molécule NP. L'invention concerne en effet toutes molécules adaptées à la modification de la molécule NP dans le cadre de la mise en oeuvre du procédé de modification selon l'invention, dans la mesure où le dérivé NP obtenu est dépourvu de caractère toxique, et n'a pas perdu sa capacité a être reconnu par les anticorps anti-NP.

La compatibilité du groupe de modification avec la capacité de la molécule NP à être reconnue par des anticorps anti-NP peut être vérifiée par un test in vitro comprenant la mise en contact de la molécule NP modifiée étudiée , avec des anticorps anti-NP préalablement immobilisés sur un support solide insoluble dans des conditions permettant la formation éventuelle d'un complexe immunologique entre des anticorps anti-NP et la molécule NP modifiée étudiée, et la détection de ce complexe immunologique.

L'invention concerne aussi des compositions pharmaceutiques pour le traitement de maladies virales ou de maladies autoimmunes, induites par un agent pathogène dont l'interaction avec les cellules de l'hôte peut être affectée dans le sens de la réduction de la pathogénicité de l'agent pathogène à l'égard de ces cellules en présence d'anticorps anti-NP ou de NP détoxifiés, caractérisée en ce qu'elle contient des anticorps anti-NP ou des NP détoxifiés en association avec un véhicule pharmaceutiquement acceptable et sous une dose efficace pour modifier les conditions d'interaction entre les cellules sensibles de l'hôte auquel la composition pharmaceutique est destinée et l'agent pathogène pour cet hôte.

Dans un mode de réalisation préféré de la composition pharmaceutique selon l'invention, le groupe chimique de modification au niveau du C1 du cycle phényle ou de la position phénol du NP est un acide aminé, notamment la glycine ou la lysine, un peptide ou une protéine, notamment la BSA, l'OVA ou la myosine.

D'une façon générale, la modification d'une molécule NP par le groupe choisi peut être réalisée par toute réaction chimique appropriée mettant en oeuvre la position C1 du cycle phényle ou la position phénol du NP, d'une part, et une fonction réactive portée par la molécule destinée à former le groupe de modification en vue de réaliser leur couplage covalent. Par exemple, la molécule de modification porte une fonction amine, cas dans lequel on peut avoir recours à toute technique appropriée pour coupler entre elles cette fonction amine avec la fonction hydroxyle du NP. Dans un autre exemple, la molécule de modification portant une fonction carboxyle on peut procéder à une réaction d'estérification.

Par exemple, les agents de condensation peuvent être constitués par des carbodiimides lorsque les fonctions complémentaires sont respectivement constituées par des groupes carboxyle et amine, ou vice-versa, par exemple le p-toluène-sulfonate de N-cyclohexyl-N-$\beta$-(N-méthyl-morpholino-éthyl)-carbodiimide et le chlorhydrate de (3-méthyl)-aminopropyl-carbodiimide ou le dicyclohexyl-carbodiimide. La réaction ester alors conduite dans des conditions usuelles dans la chimie des protéines.

Les agents de condensation peuvent encore être constitués par un 6-maléimido-caproïque-acyl-N-hydroxysuccinimide-ester ou le N-succinimidyl-3(2-pyridyldithio)-propionate (SPDP), lorsque les fonctions complémentaires sont respectivement, au départ, un groupe sulfhydryle et un groupe amine ou vice-versa.

Des conditions de réactions appropriées sont encore celles décrites dans les articles suivants : J. CARLSSON et coll. Biochem. J. (1978) 173, 727-737 ou P.E. THORPE et coll. dans Euro. J. Biochem. (1981), 116, 447-454.

L'invention concerne en outre l'application d'une composition pharmaceutique décrite ci-dessus, à la fabrication d'un médicament pour le traitement d'une infection virale ou d'une maladie autoimmune.

Les doses de compositions pharmaceutiques selon l'invention, administrées pour traiter des maladies virales ou des maladies autoimmunes devront être choisies de façon à ce que l'on observe l'efficacité de ces compositions dans le traitement de la maladie. Elles pourront être modulées au cours du traitement en fonction de la nature de l'évolution de l'interaction entre l'agent pathogène et les cellules sensibles.

D'autres caractéristiques techniques de l'invention apparaîtront dans les exemples illustratifs qui suivent. Ces exemples ne sont en rien limitatifs pour ce qui concerne l'application des procédés selon l'invention aux maladies virales ou autoimmunes.

## EXEMPLE I

### Effet des anticorps naturels et du TNP sur la prolifération des lymphocytes de souris

#### a/ Préparation des anticorps

Les anticorps utilisés sont des anticorps monoclonaux (AM) naturels (N5-12, N5-17, MO-1, 62-1, D-23) et proviennent d'hybridomes préparés par fusion des cellules de rate de souris n'ayant reçu aucune immunisation au préalable ni aucune stimulation mitogénique, avec le myélome de souris X63-Ag8. Les hybridomes TNP11 et TNP9 proviennent de fusion des cellules de rate de souris immunisées avec du Ficoll-TNP. Ils ont été maintenus en culture dans le milieu RPMI complet contenant 10% de sérum de veau foetal. Les hybridomes ont été injectés à des souris Balb/c préalablement traitées au pristane dans le but d'obtenir des ascites. Les AM ont été utilisés en tant que surnageants de culture ou en tant qu'ascites ou après isolement par chromatographie d'affinité sur une colonne de DNP-Sépharose. Ces anticorps ont été testés par test immunoenzymatique sur différents antigènes immobilisés sur des plaques de polystyrène et révélés par un anticorps anti-Ig de souris couplé à la $\beta$-galactosidase.

#### b/ Préparation des dérivés NP

Le TNP a été couplé à la sérumalbumine bovine (BSA) par la méthode de Little & Eisen : 1ml de BSA à 20mg/ml dans le tampon carbonate-bicarbonate, 0,1M, pH 9,5, est ajouté à 1ml d'une solution d'acide trinitrobenzène sulfonique à 2 mg/ml dans le même tampon. La réaction est arrêtée après 30 à 120 minutes selon la substitution voulue par élimination de l'excès de réactifs sur échangeur d'ions (Dowex 1 x 4) puis dialyse. Des préparations de BSA substituée avec soit 14 ou 30 molécules de TNP par molécule de BSA ($TNP_{14}$-BSA ou $TNP_{30}$-BSA) ont été utilisées.

Le DNP-Glycine (DNP-Gly) et le DNP-Lysine (DNP-Lys) proviennent des Laboratoires Sigma et sont solubilisés directement dans le milieu de culture.

#### c/ Préparation des populations cellulaires

Les cellules proviennent de rate ou de thymus de souris. Les souris sont tuées par dislocation cervicale et leur rate ou leur thymus prélevé stérilement sont déposés dans du milieu RPMI. Les cellules sont dissociées, lavées une fois avec du milieu puis les globules rouges sont lysés au chlorure d'ammonium (0,9%) pendant 1 minute à froid puis les cellules sont relavées dans le milieu complet.

Les sous-populations de cellules B ou T sont ensuite séparées comme suit :
- Sous-population T : les cellules T sont obtenues à partir de la population de cellules de la rate par élimination des cellules portant des Ig à leur surface (sous-population B) sur des anticorps anti-Ig de souris immobilisés soit sur des billes magnétiques (Magnogel)® soit sur des boites de Pétri.
- Sous-population B : les cellules B sont obtenues après détachement mécanique des supports dur lesquels elles étaient fixées par l'intermédiaire des anticorps anti-Ig (après l'isolement des cellules T), ou après élimination de la sous-population des cellules T de la population totale des cellules de rate par un traitement avec un anticorps anti-cellules T suivi de complément de cobaye.
- Thymocytes : les thymocytes sont dissociés dans le RPMI et les cellules isolées sont lavées dans le milieu complet avant d'être mises en culture.

#### d/ Culture des cellules

Le milieu de culture utilisé est du RPMI 1640 contenant du sérum de veau foetal (5%), du pyruvate de sodium (1mM), de la L-glutamine (1mM), du 2-mercaptoethanol (0,05 mM), de la pénicilline (100$\mu$g/ml) et de la streptomycine (50$\mu$g/ml).

$2x10^5$ cellules de chaque population cellulaire ou $4x10^5$ de cellules de rate non fractionnées sont distribuées sous un volume final de 200$\mu$l dans des boîtes de culture de 96 puits à fond plat en présence ou non des différents stimulateurs (ou mitogènes)(40$\mu$g/ml de lipopolysaccharide W Salmonella typhosa 2$\mu$g/ml de concanavaline A, ou des surnageants de culture contenant les anticorps monoclonaux à différentes dilutions ou une solution de TNP-BSA à des concentrations finales allant de 200ng à 200$\mu$g/ml). Les cultures sont effectuées pendant 48 à 96 heures à 37°C dans une atmosphère de 5% de CO2.

e/ Mesure de la prolifération des cellules

Six à huit heures avant la fin de la culture, 100$\mu$l de surnageant sont prélevés de chaque puits de culture afin d'être testés quant à leur contenu en anticorps. 10$\mu$l d'une solution de $^3$H-thymidine (activité spécifique = 5uCi = 185GBq/mM) à 50 $\mu$Ci/ml sont ajoutés à la culture qui est ensuite poursuivie. Les cellules de chaque puits sont collectées sur du papier filtre à l'aide d'un collecteur automatique et, après sèchage, les filtres sont comptés dans un liquide scintillant.

Les résultats obtenus sont rapportés dans le tableau I pour ce qui concerne l'action des anticorps monoclonaux sur des cellules données et dans le tableau II pour ce qui a trait à l'action des dérivés NP.

## TABLEAU I

### Effet des anticorps monoclonaux sur la prolifération des lymphocytes de la souris.

Le pourcentage d'augmentation (+) ou d'inhibition (-) est calculé par rapport aux chiffres obtenus avec le milieu de culture seul.

| Anti-corps | D23 | 62-1 | TNP11 | TNP9 |
|---|---|---|---|---|
| **Cellules de rate** | | | | |
| 1/10 | + 20% | - 90% | - 90% | O |
| 1/40 | + 60% | - 48% | - 80% | O |
| 1/160 | + 20% | - 26% | - 10% | + 40% |
| 1/640 | O | + 40% | + 40% | + 40% |
| **Cellules B** | | | | |
| 1/10 | + 10% | - 90% | + 92% | - 52% |
| 1/40 | + 300% | - 80% | + 42% | - 10% |
| 1/160 | + 300% | - 46% | + 28% | + 42% |
| 1/640 | O | + 100% | O | - |
| **Cellules T** | | | | |
| 1/10 | + 1000% | + 700% | + 2000% | - |
| 1/40 | + 500% | + 600% | + 700% | - |
| 1/160 | + 400% | + 500% | + 500% | - |
| 1/640 | O | - | - | - |
| **Thymocytes** | | | | |
| 1/10 | + 100% | O | + 400% | + 200% |
| 1/40 | O | O | + 200% | O |
| 1/160 | O | O | + 100% | O |

TABLEAU II

Effets des dérivés NP sur la prolifération des lymphocytes de la souris.

Le pourcentage d'augmentation (+) ou d'inhibition (-) est calculé par rapport aux chiffres obtenus avec le milieu seul (M) ou additionné de lipopolysaccharide (LPS) ou de concavaline (con A).

*Après traitement des cellules avec les dérivés NP pendant 48 heures, les mitogènes concanavaline A (con A) et lipopolysaccharide (LPS) sont ajoutés aux cellules et la culture poursuivie pendant 24 à 48 heures.

| Dérivés NP | TNP-BSA | | | DNP-Glycine | | | TNP-Lysine | | |
|---|---|---|---|---|---|---|---|---|---|
| | M | conA* | LPS* | M | conA* | LPS* | M | conA* | LPS* |
| **Cellules de rate** | | | | | | | | | |
| 200µg/ml | +50% | +46% | +30% | -64% | -50% | -60% | 0 | -6% | -3% |
| 50 | +30% | +30% | +20% | -30% | 0 | -20% | 0 | 0 | 0 |
| 12 | +10% | +28% | 0 | -10% | -30% | 0 | 0 | 0 | 0 |
| **Cellules B** | | | | | | | | | |
| 200µg/ml | +100% | nt | +18% | -90% | nt | -80% | -50% | nt | nt |
| 50 | +50% | nt | 0 | -60% | nt | -64% | -30% | nt | nt |
| 12 | 0 | nt | 0 | -50% | nt | -40% | -50% | nt | nt |
| 3 | 0 | nt | 0 | -25% | nt | -7% | -40% | nt | |
| **Cellules T** | | | | | | | | | |
| 200µg/ml | +30% | -50% | nt | +4% | -45% | nt | +32% | -50% | nt |
| 50 | +18% | -25% | nt | +30% | -15% | nt | +20% | -20% | nt |
| 12 | +20% | -20% | nt | 0 | -4% | nt | 0 | -20% | nt |
| 3 | 0 | -40% | nt | 0 | -5% | nt | 0 | -10% | nt |
| 0,7 | 0 | -50% | nt | +50% | 0 | nt | 0 | 0 | nt |

CONCLUSIONS

Selon les anticorps monoclonaux et selon leur concentration (approximativement de 1 à 100 μg par ml) les effets observés sont positifs ou négatifs. En outre, les effets du LPS sur les cellules B sont significativement inhibés en présence de concentrations élevées d'anticorps monoclonal.

Ces résultats démontrent l'existence d'une activité des anticorps anti-NP et des dérivés NP sur la prolifération des cellules lymphocytaires. Les cellules lymphocytaires sont donc parmi les cellules sensibles qui peuvent être visées par les procédés selon l'invention.

EXEMPLE II

Effet vironeutralisant in vitro des anticorps anti-NP et des dérivés NP sur l'infectivité du virus de la chorioméningite lymphocytaire (CML).

L'effet des anticorps anti-NP en surnageant de culture et des dérivés NP a été étudié sur le CML in vitro, en testant la réduction des plages des cellules infectées.

Préparation du milieu de culture.

Le mélange méthyl cellulose-Dulbecco est obtenu comme suit :
- Préparation de la solution de méthyl cellulose : on dissout 1,8g de méthyl cellulose dans 100 ml d'eau distillée sous agitation magnétique continue à +4°C pendant 2 jours. Ensuite, on stérilise à 105°C pendant 20 minutes et on conserve à la température du laboratoire.
- Préparation du milieu de Dulbecco : on dissout la poudre d'un sachet prévu pour 5 litres de milieu dans 2 litres et demi d'eau distillée puis on ajoute 2,2g de bicarbonate de sodium par litre de milieu et on stérilise par filtration sur filtre 0,22 $\mu$m.

Préparation du violet cristallisé.

- On ajoute 1 volume de formol et 8 volumes d'eau distillée ajoutés à 1 volume de solution mère.
- Solution mère : violet cristallisé 20g, éthanol 200 ml formol 100 ml, eau distillée 700 ml.

Technique des plages pour le virus CML

24 heures avant l'infection, on met en culture après trypsination des cellules L929 Gibco®, qui sont en fin de "lag phase", (18-24 h après la mise en culture). La suspension cellulaire est ajustée à $0,5x10^5$ cellules par ml dans du milieu Dulbecco (dont la préparation est donnée dans la suite) plus 3,7g de bicarbonate de sodium par litre et 5% de sérum de veau foetal. Les cellules sont réparties à raison de 2ml par cupule et elles sont incubées à 37°C en atmosphère de 10% de $CO_2$ pendant au maximum 24 heures avant l'infection.

1 heure avant l'infection, les cellules sont mises à 37°C en présence de l'anticorps ou de l'antigène dilué dans le milieu cellulaire, juste avant l'infection on élimine le milieu de culture contenant l'anticorps ou l'antigène des cupules et on distribue le virus (pfu/ml) à raison de 0,5ml par cupule. Une cupule est gardée comme témoin des cellules et reçoit 0,5ml de milieu et une cupule qui n'a pas reçu l'anticorps ou l'antigène reçoit le virus et elle est considérée comme témoin virus. On incube les cellules à 37°C pendant une heure à 10% de $CO_2$ en présence du virus. On élimine l'inoculum et on ajoute dans chaque cupule 2ml du mélange méthyl cellulose-Dulbecco (1 volume de méthyl cellulose + 1 volume de milieu de Dulbecco contenant 10% de sérum de veau foetal).

On incube la plaque pendant 5 jours à 37°C en atmosphère de 5% de $CO_2$. On élimine le milieu des cupules par retournement de la plaque. On rince 3 fois avec du PBS et on colore pendant 20 minutes avec une solution de violet cristallisé.

Finalement, on rince à l'eau du robinet et on compte les plages.

Les résultats de ces essais sont rapportés dans la suite (tableaux III à XIII).

## TABLEAU III

### Neutralisation de l'activité lytique du CML par les AcN et les anticorps anti-NP

Séroneutralisation : Aucun effet

### Neutralisation de l'activité lytique du CML par les AcN et les anticorps anti-NP préincubés avec les cellules L.929

|  | % Inhibition |
|---|---|
| Témoin virus | 0 |
| Anticorps TNP11 en surnageant pur | 53 |
| " 1/2 | 47 |
| " 1/4 | 50 |
| Anticorps TNP9 en surnageant pur | 61 |
| " 1/2 | 44 |
| " 1/4 | 44 |
| Anticorps 621 en surnageant pur | 22 |
| " 1/2 | 22 |
| " 1/4 | 20 |

(Les anticorps D23 et mo1 donnent des résultats comparables à ceux obtenus avec le 621).

## TABLEAU IV

### Neutralisation de l'activité lytique du CML par les dérivés NP

### Séroneutralisation

|  | % Inhibition |
|---|---|
| Témoin virus | 0 |
| TNP-BSA à 1mg/ml | 96 |
| "         0.5 " | 92 |
| "         0.25" | 78 |
| "         0.12" | 61 |
| "         0.06" | 41 |
| "         0.03" | 32 |
| DNP-glycine à 0.48 mg/ml | 86 |
| "         0.240       " | 90 |
| "         0.120       " | 80 |
| "         0.060       " | 77 |
| "         0.030       " | 48 |
| "         0.015       " | 41 |

TABLEAU V

| Neutralisation de l'activité lytique du CML par les dérivés NP préincubés avec les cellules L929 | |
|---|---|
|  | % Inhibition |
| Témoin virus | 0 |
| TNP-BSA à 1 mg/ml | 63 |
| TNP-BSA à 0.5 mg/ml | 48 |
| TNP-BSA à 0.25 mg/ml | 22 |
| TNP-BSA à 0.12 mg/ml | 0 |
| DNP-glycine à 0.48 mg/ml | 55 |
| DNP-glycine à 0.24 mg/ml | 25 |
| DNP-glycine à 0.12 mg/ml | 30 |
| DNP-glycine à 0.06 mg/ml | 15 |
| DNP-glycine à 0.03 mg/ml | 80 |
| DNP-glycine à 0.01 mg/ml | 25 |

TABLEAU VI

| Neutralisation de l'activité lytique du CML par les dérivés NP ajoutés sur la culture cellulaire après l'infection virale. | |
|---|---|
| | % Inhibition |
| Témoin virus | 0 |
| TNP-BSA à 1mg/ml | 17 |
| TNP-BSA à 0.5 mg/ml | 0 |
| DNP-glycine à 0.48 mg/ml | 63 |
| DNP-glycine à 0.24 mg/ml | 54 |
| DNP-glycine à 0.12 mg/ml | 36 |
| DNP-glycine à 0.06 mg/ml | 34 |
| DNP-glycine à 0.03 mg/ml | 17 |
| DNP-glycine à 0.01 mg/ml | 10 |

CONCLUSIONS

Parmi les anticorps monoclonaux de souris utilisés (TNP11, TNP9, D-23, 62-1, MO1) seuls les anticorps TNP11 et TNP9 spécifiques des déterminants TNP ou DNP peuvent neutraliser l'effet lytique du CML. L'action des anticorps anti-NP semble ne pas s'exercer directement sur les antigènes viraux. Ces anticorps semblent neutraliser l'effet viral en agissant sur la cellule. Les dérivés NP agissent en séroneutralisation et en incubation avec les cellules. Ceci montre que leur mode d'action s'exerce à la fois sur le virus et sur la cellule. Les résultats montrent que le dérivé DNP-glycine peut inhiber le virus même s'il est ajouté à la culture cellulaire après l'infection virale, probablement en pénétrant dans la cellule et en agissant directement sur le virus ou sur sa réplication.

EXEMPLE III :

Neutralisation in vitro de l'activité lytique du virus de la stomatite vésiculeuse (VSV) par l'AcN N5-17.

1/ Séroneutralisation

Le N5-17 est utilisé ici sous forme d'ascite.

On procède à une séroneutralisation classique du VSV brut $L_3$ (passé 3 fois sur cellules L929) dilué de façon à obtenir 50 plages par cupule par l'anticorps N5-17 en différentes dilutions. Le virus est incubé en présence de l'anticorps 1 heure à 37°C au bain marie. La neutralisation de l'activité lytique est testée ensuite par technique des plages sur cellules L929 (technique des plages décrites en détail pour le CML). Les plages sont révélées 48 ou 72 heures après par coloration au violet cristallisé. Les valeurs représentées dans les résultats (tableau VII) sont celles obtenues après correction pour tenir compte de l'inhibition obtenue avec des ascites témoins.

TABLEAU VII

| | % inhibition |
|---|---|
| - Témoin virus | 0 |
| - Anticorps N5-17 en ascite, pur | 58 |
| - Anticorps N5-17 en ascite, dilué au 1/2 | 48 |
| - Anticorps N5-17 en ascite, dilué au 1/4 | 32 |
| - Anticorps N5-17 en ascite, dilué au 1/8 | 26 |
| - Anticorps N5-17 en ascite, dilué au 1/16 | 20 |
| - Anticorps N5-17 en ascite, dilué au 1/32 | 10 |

EP 0 316 212 B1

2/ Neutralisation de l'activité lytique du VSV par l'anticorps N5-17 préincubé avec les cellules L.929

L'anticorps N5-17 à différentes dilutions et les cellules L929 sont incubés ensemble pendant 1 heure à 37°C dans une atmosphère enrichie en $CO_2$ ou pendant 1 heure à + 4°C. Après lavage pour éliminer l'excès de l'anticorps N5-17, on infecte avec le VSV dilué de façon à obtenir 50 plages par cupule. La neutralisation de l'effet lytique du VSV est testée par la technique des plages (décrite en détail pour le CML). Les valeurs données dans les résultats (tableau VIII) sont celles obtenues après correction pour tenir compte de l'inhibition obtenue avec des ascites témoins.

TABLEAU VIII

| Préincubation des cellules L929 avec l'anticorps N5-17 pendant 1 heure à 37°C | |
| --- | --- |
| | % Inhibition |
| - Témoin virus | 0 |
| - Anticorps N5-17 en ascite au 1/2 | 40 |
| - Anticorps N5-17 en ascite au 1/4 | 37 |
| - Anticorps N5-17 en ascite au 1/8 | 30 |
| - Anticorps N5-17 en ascite au 1/16 | 4 |
| Préincubation des cellules L929 avec l'anticorps N5-17 pendant 1 heure à 4°C | |
| | % Inhibition |
| - Témoin virus | 0 |
| - Anticorps N5-17 en ascite au 1/2 | 0 |

EXEMPLE IV

Neutralisation in vitro de l'activité lytique du VSV par l'anticorps N5-12

Séroneutralisation

Le N5-12 est utilisé ici sous forme de surnageant de culture. On procède à une séroneutralisation comme décrite pour l'anticorps N5-17 et à une révélation de la neutralisation de l'activité lytique du VSV par la méthode des plages. Les résultats sont donnés dans le tableau IX.

TABLEAU IX

| | % Inhibition |
| --- | --- |
| - Témoin virus | 0 |
| - Anticorps N5-12 en surnageant pur | 60 |
| - Anticorps N5-12 en surnageant au 1/2 | 44 |
| - Anticorps N5-12 en surnageant au 1/4 | 25 |
| - Anticorps N5-12 en surnageant au 1/8 | 6 |
| - Anticorps N5-12 en surnageant au 1/16 | 0 |
| - Anticorps TNP11 en surnageant pur | 0 |

14

Neutralisation de l'activité lytique du VSV par l'anticorps N5-12 préincubé avec les cellules L929

On procède comme décrit pour l'anticorps N5-17, et on obtient les résultats du tableau X.

TABLEAU X

| Préincubation des cellules L929 avec l'anticorps N5-12 pendant 1 heure à 37°C | |
|---|---|
| | % Inhibition |
| - Témoin virus | 0 |
| - Anticorps N5-12 en surnageant pur | 0 |
| Préincubation des cellules L929 avec l'anticorps N5-12 pendant 1 heure à 4°C | |
| | % Inhibition |
| - Témoin virus | 0 |
| - Anticorps N5-12 en surnageant pur | 0 |

CONCLUSIONS pour exemples III et IV

Les AcN polyspécifiques peuvent neutraliser l'effet lytique du virus de la stomatite vésiculeuse en réagissant avec des épitopes viraux et en séroneutralisant ainsi le virus (anticorps N5-17, anticorps N5-12) ou bien en réagissant avec des épitopes cellulaires en relation avec la fixation et la pénétration du virus dans les cellules (anticorps N5-17).

EXEMPLE V :

Vironeutralisation du virus HIV1 in vitro par les anticorps anti-NP et les dérivés NP

La technique de culture et de titrage de la reverse-transcriptase du HIV est rappelée ci-après : Des lymphocytes humains, préalablement activés par la PHA (phytohémagglutinine) sont incubés dans un milieu nutritif (RPMI 1640 + sérum de veau foetal + antibiotiques + $\beta$-mercaptoéthanol $10^{-5}$ M + 2$\mu$g/ml polybrene + sérum anti-interféron 1/2000 + IL2 10%). Après centrifugation, le culot cellulaire est mis en présence du HIV1 (10.000 cpm/$10^6$ cellules) pendant 1 heure à 37°. Après lavage et centrifugation, les cellules sont resuspendues dans du milieu nutritif. Les surnageants de culture cellulaire sont suivis pendant plusieurs jours pour leur activité reverse transcriptase. Ils sont centrifugés 7 minutes à 95000 tours et le culot est lysé par du Triton® 0,1% (produit Touzart et Matignon). Un mélange réactionnel est ajouté (Tris, DTT (dithiothreitol), $MgCl_2$, KCl, PolyA, Oligo dT, $^3$HTTP, $H_2O$) et après 1 heure d'incubation à 37°C, la réaction est arrêtée avec du pyrophosphate de sodium et de l'acide nucléique de levure est ajouté pour aider la précipitation. De l'acide trichloracétique (TCA) est ajouté et la solution est laissée 15 minutes à 4°C. Après filtration sous vide avec des filtres millipores 0.45 $\mu$m et plusieurs lavages avec du TCA (0.5%), on procède au rinçage à l'éthanol, au sèchage en étuve 37° et enfin au comptage.

Séroneutralisation

Le HIV1 est incubé 1 heure à 37° en présence d'AcN (D23, 621, N5-12), d'anticorps anti-NP (TNP11) ou en présence des dérivés NP (DNP-glycine et DNP-lysine). L'activité reverse transcriptase du HIV1 est testée immédiatement (cas des dérivés DNP-glycine et DNP-lysine). Alternativement, des lymphocytes humains sont infectés par le HIV1 préincubé avec les produits à tester, et la culture cellulaire est observée pendant 3 semaines par titrage de la reverse transcriptase. Durant cette période les cultures des lymphocytes humains ne contiennent pas d'anticorps ni d'antigène.

Les résultats suivants ont été obtenus :

TABLEAU XI

| Activité reverse transcriptase dans les surnageants de culture des lymphocytes infectés avec du HIV1 préincubé avec des anticorps. | | | | | |
|---|---|---|---|---|---|
| temps en jours après l'infection | 3 | 7 | 10 | 14 | 17 |
| Témoin virus | 792 | 16130 | 58260 | 14610 | 8820 |
| N5-12 | 4430 | 6498 | 17482 | 21958 | 2602 |
| D23 | 1768 | 6386 | 18020 | 55392 | 3804 |
| 621 | 1918 | 8320 | 15070 | 13112 | 2605 |
| TNP11 | 1018 | 5138 | 11755 | 19638 | 9504 |

## TABLEAU XII

Activité reverse transcriptase du virus HIV1 incubé avec des dérivés NP et testé immédiatement pour l'activité enzymatique.

Activité reverse transcriptase

| | | |
|---|---|---|
| Témoin virus | | 11924 |
| DNP-glycine | $10^{-3}$M | 5444 |
| " | $10^{-6}$M | 3424 |
| " | $10^{-7}$M | 4090 |
| " | $10^{-9}$M | 3638 |
| DNP-lysine | $10^{-3}$M | 4652 |
| " | $10^{-6}$M | 3932 |
| " | $10^{-7}$M | 7240 |
| " | $10^{-9}$M | 9262 |

CONCLUSIONS

Lorsque le HIV1 est préincubé en présence d'AcN et d'anticorps anti-NP aucune différence significative de l'activité reverse transcriptase, par rapport au témoin virus, n'a été retrouvée. Lorsque le HIV1 est préincubé avec des dérivés NP on observe une nette séroneutralisation. En présence de DNP-glycine, on observe une séroneutralisation du virus qui est valable pour des concentrations allant de $10^{-3}$ à $10^{-9}$M, et en présence de DNP-lysine la séroneutralisation est observée jusqu'à la concentration du produit $10^{-6}$M.

2/ Neutralisation de l'activité enzymatique du HIV1 par les anticorps anti-NP et les dérivés NP, préincubés avec les lymphocytes.

Les lymphocytes humains, préalablement activés par la PHA, sont incubés en présence des AcN, des anticorps anti-NP et des dérivés NP (DNP-glycine, DNP-lysine) pendant une nuit à 37°C. Le lendemain, le culot cellulaire est mis en présence du HIV1 pendant 1 heure à 37°C au bain-marie et les cellules sont resuspendues dans du milieu nutritif en présence des produits à tester. Les surnageants de culture sont testés pour l'activité reverse transcriptase pendant 3 semaines.

**T A B L E A U   XIII**

Temps en jours après l'infection.

| | 3 | 6 | 10 | 13 | 18 | 20 | 24 | 26 | 31 |
|---|---|---|---|---|---|---|---|---|---|
| Témoin virus | 1384 | 12494 | 75072 | 56426 | 1224 | 0 | 0 | - | - |
| D 23 | 15 | 16082 | 33065 | 8008 | 7016 | 508 | 3032 | - | - |
| TNP 9 | 0 | 0 | 0 | 0 | 0 | 0 | 0 | 0 | 0 |
| Témoin virus | 3290 | 5128 | 263836 | 11290 | 8444 | 1170 | - | - | - |
| TNP 11 50µg/ml | 2156 | 0 | 0 | 0 | 0 | 29548 | 125380 | 23952 | - |
| TNP 11 12µg/ml | 618 | 298 | 476 | 214 | 0 | 56 | 212 | 0 | - |
| Témoin virus | 562 | 1274 | 94474 | 63944 | 73368 | 26840 | | | |
| DNP-lysine | 936 | 958 | 1102 | 1466 | 958 | 1268 | | | |
| DNP-glycine | 1064 | 890 | 1234 | 1572 | 1276 | 1270 | | | |

## CONCLUSIONS

L'incubation des lymphocytes avec des AcN avant ou pendant l'addition du virus ne modifie pas de façon significative, par rapport au témoin virus, l'activité reverse transcriptase.

Les anticorps anti-NP (du type IgM (TNP-11) et du type IgG (TNP9)) retardent l'apparition de l'activité reverse transcriptase par rapport au témoin virus ou inhibent complètement l'apparition de l'activité enzymatique virale. Cet effet est dose dépendante et varie selon le temps de l'addition de l'anticorps dans la culture. Les dérivés NP (DNP-glycine et DNP-lysine) peuvent conduire à une inhibition totale de l'infectivité virale et cette inhibition ne nécessite pas la présence continue de ces dérivés à la culture des lymphocytes infectés.

EXEMPLE VI : EFFET DE NP CHIMIQUEMENT MODIFIES SUR LA PROLIFERATION DE VIRUS

L'effet des NP couplés (TNP-BSA, DNP-Glycine) a été évalué par la méthode des plages, comme pour le virus de la chorioméningite lymphocytaire dans l'exemple I. L'effet neutralisant des NP couplés a été évalué sur les virus enveloppés ou non enveloppés suivants :

1) Virus enveloppés :

IHDT-6 (International Health Division-Tumor-6):

Ce virus est un Pox Virus à ADN double brin. On l'a cultivé sur des cellules de souris TCC31. Les résultats ont été obtenus après 3 jours de culture.

VSV (Vesicular Stomatitis Virus)

Il s'agit d'un Rhabdovirus à simple brin d'ARN. Il est mis en culture sur cellules L929 de souris. Les résultats sont obtenus après 3 jours de culture.

HVE (Herpes Virus Eidolon)

C'est un virus de l'herpès à double brin d'ADN que l'on cultive sur des cellules TCC31 de souris. Les résultats sont observés après 2 Jours de culture.

2) Virus non enveloppés :

EMCV (Encephalomyocarditis Virus)

Ce Picorna-virus à ARN simple brin a été cultivé sur cellules L929 de souris. Les résultats sont obtenus après 1 jour de culture.

Pour tous les virus testés, les dilutions des virus donnaient 50 plages/cupule.

RESULTATS

<u>IHDTV</u>

| TNP-BSA conc.en mg/ml | % Inhibition |
|---|---|
| 2,4 | 72 |
| 1,2 | 73 |
| 0,6 | 51 |
| 0,3 | 37 |
| 0,15 | 31 |
| 0,07 | 21 |

<u>Conclusion</u> : Le TNP-BSA peut séroneutraliser le virus IHD jusqu'à une concentration de 0,6 mg/ml.

<u>HVE</u>

| TNP-BSA conc.en mg/ml | % Inhibition | DNP-Glyc conc.en mg/ml | % Inhibition |
|---|---|---|---|
| - | - | 2,4 | 92 |
| 1,0 | 79 | 1,2 | 57 |
| 0,5 | 55 | 0,6 | 50 |
| 0,25 | 35 | 0,3 | 48 |
| 0,12 | 16 | 0,15 | 40 |
| 0,06 | 5 | 0,07 | 28 |
| 0,03 | 0 | 0,03 | - |

Conclusion : Le TNP-BSA peut sénoneutraliser le virus HVE jusqu'à une concentration de 0,5 mg/ml. Le DNP-Glycine peut également séroneutraliser le virus HVE jusqu'à une concentration de 0,6 mg/ml.

<u>VSV</u>

| TNP-BSA conc.en mg/ml | % Inhibition | DNP-Glyc conc.en mg/ml | % Inhibition |
|---|---|---|---|
| 2,0 | 24 | 2,4 | 41 |
| 1,0 | 15 | 1,2 | 68 |
| 0,5 | 12 | 0,6 | 47 |
| 0,25 | O | 0,3 | 38 |
| 0,12 | O | 0,15 | 6 |
| 0,06 | O | 0,07 | O |
| 0,03 | O | 0,03 | O |

Conclusion : Le TNP-BSA ne séroneutralise pas le virus VSV. Le DNP-Glycine a un effet jusqu'à une concentration de 1,2 mg/ml.

EMCV

| TNP-BSA conc.en mg/ml | % Inhibition | DNP-Glyc conc.en mg/ml | % Inhibition |
|---|---|---|---|
| 2,0 | 14 | - | - |
| 1,0 | 4 | 1,2 | 78 |
| 0,5 | 8 | 0,6 | 62 |
| 0,25 | 0 | 0,3 | 32 |
| 0,12 | 0 | 0,15 | 20 |
| 0,06 | 0 | 0,07 | 0 |
| 0,03 | 0 | 0,03 | 0 |

Conclusion : Le TNP-BSA n'a aucun effet neutralisant sur EMCV. Le DNP-Glycine séroneutralise jusqu'à une concentration de 0,6 mg/ml.

**Revendications**

1. Procédé pour modifier in vitro les conditions de l'interaction entre les cellules sensibles d'un hôte vivant et un agent pathogène pour cet hôte, caractérisé par la mise en contact de ces cellules sensibles à cet agent pathogène, avec des anticorps anti-nitrophényles, ou avec des molécules contenant un cycle benzénique dont un, deux ou trois atomes d'hydrogène est ou sont substitués par un groupe nitro, chimiquement modifiées par l'intermédiaire d'une liaison covalente au niveau du C1 du cycle phényle ou au niveau de la position phénol du nitrophényle, par un groupe chimique de neutralisation des éventuelles propriétés toxiques in vivo desdites molécules non modifiées, sans néanmoins affecter leur capacité à être reconnues par les anticorps anti-nitrophényles, et ce dans des conditions, notamment de dosage des anticorps anti-nitrophényles ou desdits nitrophényles modifiés, fonction du degré de modulation recherché de la susdite interaction.

2. Procédé selon la revendication 1, caractérisé en ce que l'agent pathogène est un virus.

3. Procédé selon l'une quelconque des revendications 1 ou 2, caractérisé en ce que les cellules sensibles sont mises en contact avec des molécules contenant un cycle benzénique dont un, deux ou trois atomes d'hydrogène est ou sont substitués par un groupe nitro, chimiquement modifiées par tout groupe chimique détoxifiant n'interférant et ne modifiant pas défavorablement l'influence exercée in vitro par les nitrophényles sur la susdite interaction.

4. Procédé selon l'une quelconque des revendications 1 à 3, caractérisé en ce que les cellules sensibles sont mises en contact avec des molécules contenant un cycle benzénique dont un, deux ou trois atomes d'hydrogène est ou sont substitués par un groupe nitro, chimiquement modifiées par un acide aminé, ou un peptide.

21

**5.** Procédé selon la revendication 4, caractérisé en ce que le nitrophényle est chimiquement modifié par un acide aminé et en ce que l'acide aminé est la glycine ou la lysine.

**6.** Procédé selon l'une quelconque des revendications 1 à 3, caractérisé en ce que le nitrophényle est chimiquement modifié par une protéine.

**7.** Procédé selon la revendication 6, caractérisé en ce que la protéine est la BSA.

**8.** Composition pharmaceutique pour le traitement de maladies virales ou de maladies autoimmunes, induites par un agent pathogène, dont l'interaction avec les cellules de l'hôte peut être affectée dans le sens de la réduction de la pathogénicité de l'agent pathogène à l'égard de ces cellules, caractérisée en ce qu'elle contient des anticorps anti-nitrophényles ou des molécules contenant un cycle benzénique dont un, deux ou trois atomes d'hydrogène est ou sont substitués par un groupe nitro, chimiquement modifiées, par l'intermédiaire d'une liaison covalente au niveau du C1 du cycle phényle ou au niveau de la position phénol du groupe nitrophényle, par un groupe chimique de neutralisation des éventuelles propriétés toxiques in vivo desdites molécules non modifiées, sans néanmoins affecter leur capacité à être reconnues par les anticorps anti-nitrophényles, en association avec un véhicule pharmaceutiquement acceptable, et sous une dose efficace pour modifier les conditions d'interaction entre les cellules sensibles de l'hôte auquel la composition pharmaceutique est destinée et l'agent pathogène pour cet hôte.

**9.** Composition pharmaceutique selon la revendication 8, caractérisée en ce que les molécules contenant un cycle benzénique dont un, deux ou trois atomes d'hydrogène est ou sont substitués par un groupe nitro, sont chimiquement modifiées par tout groupe chimique détoxifiant n'interférant et ne modifiant pas défavorablement l'influence exercée in vitro par les nitrophényles sur la susdite interaction.

**10.** Composition pharmaceutique selon la revendication 8 ou 9, caractérisée en ce que les molécules contenant un cycle benzénique dont un, deux ou trois atomes d'hydrogène est ou sont substitués par un groupe nitro, sont chimiquement modifiées par un acide aminé, ou un peptide, en particulier en ce que l'acide aminé est la glycine ou la lysine.

**11.** Composition pharmaceutique selon l'une quelconque des revendications 8 ou 9, caractérisée en ce que les molécules contenant un cycle benzénique dont un, deux ou trois atomes d'hydrogène est ou sont substitués par un groupe nitro, sont chimiquement modifiées par une protéine, par exemple la BSA.

**12.** Utilisation du principe actif d'une composition pharmaceutique selon l'une des revendications 8 à 11 pour l'obtention d'un médicament destiné au traitement d'une affection virale ou d'une maladie autoimmune.

**Claims**

**1.** Process for modifying in vitro the conditions for interaction between the sensitive cells of a living host and a pathogenic agent for this host, characterized by the placing of these cells which are sensitive to this pathogenic agent in contact with anti-nitrophenyl antibodies or with molecules containing a benzene ring of which one, two or three hydrogen atoms is or are substituted by a nitro group, which molecules are chemically modified through a covalent bond at the C1 position of the phenyl ring or at the phenol position of the nitrophenyl, by a chemical group for neutralizing the possible in vivo toxic properties of the said unmodified molecules, without however affecting their capacity to be recognized by anti-nitrophenyl antibodies, and this under conditions in particular for assaying the anti-nitrophenyl antibodies or the said modified nitrophenyls, as a function of the desired degree of modulation of the abovementioned interaction.

**2.** Process according to Claim 1, characterized in that the pathogenic agent is a virus.

**3.** Process according to either of Claims 1 and 2, characterized in that the sensitive cells are placed in contact with molecules containing a benzene ring of which one, two or three hydrogen atoms is or are substituted by a nitro group, which molecules are chemically modified by any detoxifying chemical group which does not unfavourably interfere with and modify the influence exerted in vitro by the nitrophenyls on the above-mentioned interaction.

4. Process according to any one of Claims 1 to 3, characterized in that the sensitive cells are placed in contact with molecules containing a benzene ring of which one, two or three hydrogen atoms is or are substituted by a nitro group, which molecules are chemically modified by an amino acid or a peptide.

5. Process according to Claim 4, characterized in that the nitrophenyl is chemically modified by an amino acid and in that the amino acid is glycine or lysine.

6. Process according to any one of Claims 1 to 3, characterized in that the nitrophenyl is chemically modified by a protein.

7. Process according to Claim 6, characterized in that the protein is BSA.

8. Pharmaceutical composition for the treatment of viral diseases or autoimmune diseases, induced by a pathogenic agent, whose interaction with the host cells may be affected in the direction of a reduction in the pathogenicity of the pathogenic agent towards these cells, characterized in that it contains anti-nitrophenyl antibodies or molecules containing a benzene ring of which one, two or three hydrogen atoms is or are substituted by a nitro group, which molecules are chemically modified through a covalent bond at the C1 position of the phenyl ring or at the phenol position of the nitrophenyl group by a chemical group for neutralizing the possible in vivo toxic properties of the said unmodified molecules, without however affecting their capacity to be recognized by the anti-nitrophenyl antibodies, in combination with a pharmaceutically acceptable vehicle, and in a dose effective for modifying the conditions for interaction between the sensitive cells of the host for whom the pharmaceutical composition is intended and the pathogenic agent for this host.

9. Pharmaceutical composition according to Claim 8, characterized in that the molecules containing a benzene ring of which one, two or three hydrogen atoms is or are substituted by a nitro group are chemically modified by any detoxifying chemical group which does not unfavourably interfere with and modify the influence exerted in vitro by the nitrophenyls on the above-mentioned interaction.

10. Pharmaceutical composition according to Claim 8 or 9, characterized in that the molecules containing a benzene ring of which one, two or three hydrogen atoms is or are substituted by a nitro group are chemically modified by an amino acid or a peptide, in particular in that the amino acid is glycine or lysine.

11. Pharmaceutical composition according to either of Claims 8 and 9, characterized in that the molecules containing a benzene ring of which one, two or three hydrogen atoms is or are substituted by a nitro group are chemically modified by a protein, for example BSA.

12. Use of the active ingredient of a pharmaceutical composition according to one of Claims 8 to 11 for producing a medicinal product intended for the treatment of a viral condition or an autoimmune disease.

**Patentansprüche**

1. Verfahren zur in vitro Modifikation der Bedingungen der Wechselwirkung zwischen empfindlichen Zellen eines lebenden Wirts und einem Krankheitserreger für diesen Wirt, gekennzeichnet durch das Inkontaktbringen der gegen den Krankheitserreger empfindlichen Zellen mit Antinitrophenyl-Antikörpern oder mit Molekülen, die einen Benzolring enthalten, in dem ein, zwei oder drei Wasserstoffatome durch eine Nitrogruppe substituiert sind, und die chemisch über eine kovalente Bindung an der C1-Position des Phenylrings oder an der Phenolposition des Nitrophenylrests durch eine chemische Gruppe modifiziert sind, die etwaige toxische Eigenschaften dieser nichtmodifizierten Moleküle in vivo neutralisiert, ohne jedoch ihre Fähigkeit, von den Antinitrophenyl-Antikörpern erkannt zu werden, zu beeinflussen, und zwar unter Bedingungen, insbesondere der Dosierung der Antinitrophenyl-Antikörper oder der modifizierten Nitrophenylreste, die dem angestrebten Modulationsausmaß der obenerwähnten Wechselwirkung entsprechen.

2. Verfahren nach Anspruch 1, dadurch gekennzeichnet, daß der Krankheitserreger ein Virus ist.

3. Verfahren nach einem der Ansprüche 1 oder 2, dadurch gekennzeichnet, daß die empfindlichen Zellen mit Molekülen in Kontakt gebracht werden, die einen Benzolring enthalten, in dem ein, zwei oder drei Wasserstoffatome durch eine Nitrogruppe substituiert sind, und die chemisch durch beliebige entgiftende chemische Gruppen modifiziert sind, die weder ungünstig in den von den Nitrophenylresten in vitro auf die obenerwähnte Wechselwirkung ausgeübten Einfluß eingreifen noch diesen ungünstig modifizieren.

4. Verfahren nach einem der Ansprüche 1 bis 3, dadurch gekennzeichnet, daß die empfindlichen Zellen mit Molekülen in Kontakt gebracht werden, die einen Benzolring enthalten, in dem ein, zwei oder drei Wasserstoffatome durch eine Nitrogruppe substituiert sind, und die chemisch durch eine Aminosäure oder ein Peptid modifiziert sind.

5. Verfahren nach Anspruch 4, dadurch gekennzeichnet, daß der Nitrophenylrest chemisch durch eine Aminosäure modifiziert ist und daß die Aminosäure Glycin oder Lysin ist.

6. Verfahren nach einem der Ansprüche 1 bis 3, dadurch gekennzeichnet, daß der Nitrophenylrest chemisch durch ein Protein modifiziert ist.

7. Verfahren nach Anspruch 6, dadurch gekennzeichnet, daß das Protein BSA ist.

8. Arzneimittel zur Behandlung von Virus- oder Autoimmunkrankheiten, die durch einen Krankheitserreger hervorgerufen werden, dessen Wechselwirkung mit den Wirtszellen im Sinne einer Reduktion der Pathogenität des Krankheitserregers hinsichtlich dieser Zellen beeinflußt werden kann, dadurch gekennzeichnet, daß es Antinitrophenyl-Antikörper enthält oder Moleküle, die einen Benzolring enthalten, in dem ein, zwei oder drei Wasserstoffatome durch eine Nitrogruppe substituiert sind, und die chemisch über eine kovalente Bindung an der C1-Position des Phenylrings oder an der Phenolposition des Nitrophenylrests durch eine chemische Gruppe modifiziert sind, die etwaige toxische Eigenschaften dieser nichtmodifizierten Moleküle in vivo neutralisiert, ohne jedoch ihre Fähigkeit, von den Antinitrophenyl-Antikörpern erkannt zu werden, zu beeinflussen, in Verbindung mit einem pharmazeutisch verträglichen Träger und in einer Dosis, die wirksam ist zur Modifizierung der Bedingungen der Wechselwirkung zwischen den empfindlichen Wirtszellen, für die das Arzneimittel bestimmt ist, und dem Krankheitserreger für diesen Wirt.

9. Arzneimittel nach Anspruch 8, dadurch gekennzeichnet, daß die Moleküle, die einen Benzolring enthalten, in dem ein, zwei oder drei Wasserstoffatome durch eine Nitrogruppe substituiert sind, durch alle entgiftenden chemischen Gruppen chemisch modifiziert sind, die weder ungünstig in den von den Nitrophenylresten in vitro auf die obenerwähnte Wechselwirkung ausgeübten Einfluß eingreifen noch diesen ungünstig modifizieren.

10. Arzneimittel nach Anspruch 8 oder 9, dadurch gekennzeichnet, daß die Moleküle, die einen Benzolring enthalten, in dem ein, zwei oder drei Wasserstoffatome durch eine Nitrogruppe substituiert sind, chemisch durch eine Aminosäure oder ein Peptid modifiziert werden, insbesondere dadurch, daß die Aminosäure Glycin oder Lysin ist.

11. Arzneimittel nach einem der Ansprüche 8 oder 9, dadurch gekennzeichnet, daß die Moleküle, die einen Benzolring enthalten, in dem ein, zwei oder drei Wasserstoffatome durch eine Nitrogruppe substituiert sind, chemisch durch ein Protein, z.B. BSA, modifiziert sind.

12. Verwendung des Wirkstoffs eines Arzneimittels nach einem der Ansprüche 8 bis 11, zur Herstellung eines Medikaments, das zur Behandlung einer Viruserkrankung oder einer Autoimmunkrankheit bestimmt ist.